# EUROPEAN PATENT APPLICATION

(11) **EP 4 223 221 A1**
(43) Date of publication of application: **09.08.2023**
(21) Application number: 21875167.5
(22) Date of filing: 13.09.2021
(51) Int. Cl.: A61B 5/273

(54) **BIOLOGICAL SIGNAL TRANSMISSION DEVICE**

(30) Priority: 29.09.2020 JP 2020163297
(71) Applicant: Shimadzu Corporation, Nakagyo-ku, Kyoto-shi, Kyoto 604-8511 (JP); Prokidai Co., Ltd., Soraku-gun, Kyoto 619-0289 (JP)
(72) Inventor: MURATA, Koichi, Kyoto-shi, Kyoto 604-8511 (JP); FURUTA, Masafumi, Kyoto-shi, Kyoto 604-8511 (JP); SHIBATA, Kazuaki, Soraku-gun, Kyoto 619-0289 (JP)
(74) Representative: Kilian Kilian & Partner
(86) International application number: PCT/JP2021/033561
(87) International publication number: WO 2022/070865

(57) **Abstract**

A seal portion 24 is arranged between a first fixing surface 222 and a second fixing surface 311. A biasing member 25 comes into contact with a tapered surface 332 of a male terminal 331 received in a female terminal 231 and applies a force in a direction in which the male terminal 331 and the female terminal 231 are moved closer to each other. In a state where the male terminal 331 is received in the female terminal 231, the seal portion 24 is compressed by being sandwiched between the first fixing surface 222 and the second fixing surface 311, and a periphery of the male terminal 331 and the female terminal 231 becomes in a state of being sealed by the seal portion 24.

## Description

### TECHNICAL FIELD

The present invention relates to a biological signal transmission device.

### BACKGROUND ART

Conventionally, a device that measures a signal (biological signal) generated from a living body is known. Examples of the biological signal include an action potential of a heart, as well as an action potential of a muscle other than a heart, an action potentials of a brain cell, and the like. By measuring these biological signals, an electrocardiogram, an electromyogram, an electroencephalogram, or the like can be obtained.

This type of device includes an electrode attached to a living body (see, for example, Patent Documents 1 to 3 below). In Patent Document 1, a conductive gel is connected to an external device D via a spring hook. Patent Document 2 exemplifies a snap as a shape of a connector in a connecting means to the outside. In Patent Document 3, a stud is connected to a terminal portion of an electrode body in order to prevent the electrode body from falling off a holder.

### PRIOR ART DOCUMENTS

### PATENT DOCUMENTS

Patent Document 1: JP 6221195 B2
Patent Document 2: JP H5-70552 Y
Patent Document 3: JP 6181356 B2

### SUMMARY OF THE INVENTION

### PROBLEMS TO BE SOLVED BY THE INVENTION

Use of a connection portion (snap connection portion) where electrical connection is made by fitting a male terminal and a female terminal together has an advantage that a conductor can be easily connected to an electrode. On the other hand, this type of connection portion is not usually provided with a waterproof measure, and is difficult to be used in water.

For example, in a case of measuring a biological signal generated from a living body in water, an electrode is used in water. Since a connector electrically connected to an electrode attached to a living body in water is short-circuited by contact with water, a signal from the electrode cannot be normally transmitted. For this reason, in a configuration in which a biological signal is transmitted via a snap connection portion, it has not been easy to transmit a biological signal from a living body in water.

The present invention has been made in view of the above circumstances, and an object of the present invention is to provide a biological signal transmission device in which waterproof property of a connection portion between a male terminal and a female terminal in a snap connection portion is improved.

### MEANS FOR SOLVING THE PROBLEMS

Afirst aspect of the present invention is a biological signal transmission device including an electrode, a snap connection portion, a first insulator, a second insulator, and a seal portion. The electrode is attached to a living body. The snap connection portion is electrically connected to the electrode. The first insulator has a first fixing surface fixed to the electrode side of the snap connection portion. The second insulator has a second fixing surface fixed to a side opposite to the electrode side of the snap connection portion. The seal portion is arranged between the first fixing surface and the second fixing surface.

The snap connection portion includes a first connection portion, a second connection portion, and a biasing member. The first connection portion includes a male terminal on which a tapered surface is formed. The second connection portion has a female terminal that removably receives the male terminal, and is electrically connected to the first connection portion in a state where the male terminal is received in the female terminal. The biasing member comes into contact with the tapered surface of the male terminal received in the female terminal, and applies a force in a direction in which the male terminal and the female terminal are moved closer to each other.

The seal portion is sandwiched and compressed between the first fixing surface and the second fixing surface in a state where the male terminal is received in the female terminal. In this manner, the seal portion is configured to seal a periphery of the male terminal and the female terminal. Further, the seal portion is configured to release the sealing state in a state where the male terminal is removed from the inside of the female terminal.

### EFFECTS OF THE INVENTION

According to a first aspect of the present invention, in a state where the snap connection portion has the male terminal and the female terminal, and the male terminal is received in the female terminal, the seal portion is compressed by being sandwiched between the first fixing surface and the second fixing surface, and a periphery of the male terminal and the female terminal is in a state of being sealed by the seal portion. This makes it possible to prevent water from entering a connection portion between the male terminal and the female terminal from the outside.

In particular, in a state where the male terminal is received in the female terminal, the biasing member comes into contact with the tapered surface of the male terminal and applies a force in a direction in which the male terminal and the female terminal are moved closer to each other. By the above, since the seal portion sandwiched between the first fixing surface and the second fixing surface can be further compressed, waterproof property of the connection portion between the male terminal and the female terminal can be effectively improved.

### BRIEF DESCRIPTION OF THE DRAWINGS

FIG. 1 is a perspective view illustrating a configuration example of a biological signal transmission device according to a first embodiment.
FIG. 2 is a side view of the biological signal transmission device of FIG. 1.
FIG. 3 is a perspective view of a female terminal.
FIG. 4 is a cross-sectional view around a snap connection portion.
FIG. 5A is a schematic cross-sectional view for explaining a mode of snap connection by the snap connection portion, and illustrates a state before two connection portions are electrically connected.
FIG. 5B is a schematic cross-sectional view for explaining a mode of snap connection by the snap connection portion, and illustrates a state in which two connection portions are electrically connected.
FIG. 6A is a schematic cross-sectional view for explaining a variation of an insulator, illustrating a state before two connection portions are electrically connected.
FIG. 6B is a schematic cross-sectional view for explaining the variation of the insulator, illustrating a state in which two connection portions are electrically connected.
FIG. 7A is a schematic cross-sectional view for explaining a first variation of a seal portion, illustrating a state before two connection portions are electrically connected.
FIG. 7B is a schematic cross-sectional view for explaining the first variation of the seal portion, illustrating a state in which two connection portions are electrically connected.
FIG. 8A is a schematic cross-sectional view for explaining a second variation of the seal portion, illustrating a state before two connection portions are electrically connected.
FIG. 8B is a schematic cross-sectional view for explaining the second variation of the seal portion, illustrating a state in which two connection portions are electrically connected.
FIG. 9A is a schematic cross-sectional view for explaining a third variation of the seal portion, illustrating a state before two connection portions are electrically connected.
FIG. 9B is a schematic cross-sectional view for explaining the third variation of the seal portion, illustrating a state in which two connection portions are electrically connected.
FIG. 10 is a schematic diagram illustrating a configuration example of the biological signal transmission device according to a second embodiment.

### MODE FOR CARRYING OUT THE INVENTION

### 1. First embodiment

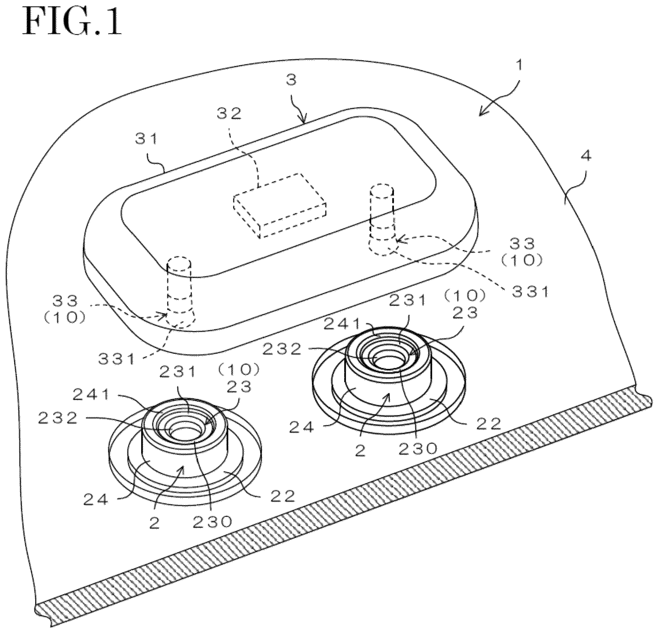

FIG. 1 is a perspective view illustrating a configuration example of a biological signal transmission device 1 according to a first embodiment. The biological signal transmission device 1 according to the present embodiment is a device for measuring a signal (biological signal) generated from a living body. A type of the biological signal is not limited as long as the biological signal is a signal detected from a living body, and examples of the biological signal include an action potential of a heart, an action potential of a muscle other than a heart, and an action potential of a brain cell. By acquiring these biological signals, an electrocardiogram, an electromyogram, an electroencephalogram, or the like can be obtained as a temporal change in potential.

The biological signal transmission device 1 includes an attachment portion 2 attachable to and detachable from a living body and a main body 3 attachable to and detachable from the attachment portion 2. In the present embodiment, two of the attachment portions 2 are provided, and the attachment portions 2 are electrically connected to the common main body 3.

In the example of FIG. 1, two of the attachment portions 2 are held by a common holding member 4. The holding member 4 is a member for facilitating attachment of each of the attachment portions 2 to a living body, and may be, for example, a band or a belt attached to the living body. In FIG. 1, only a part of the holding member 4 is illustrated. However, the holding member 4 may be omitted, and each of the attachment portions 2 may be configured to be directly held by a living body.

### 2. Overall configuration of biological signal transmission device

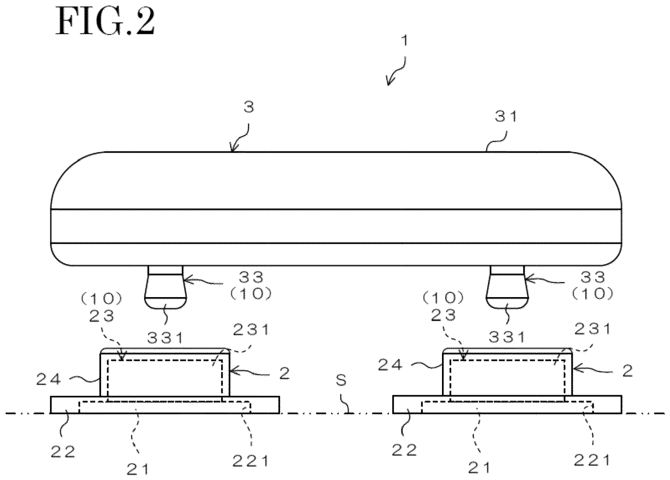

FIG. 2 is a side view of the biological signal transmission device 1 of FIG. 1. Note that, in FIG. 2, the holding member 4 is omitted from illustration. Hereinafter, an overall configuration of the biological signal transmission device 1 will be described with reference to FIGS. 1 and 2. Note that, in description below, the upper side in FIG. 2 is defined as an upper direction, and the lower side is defined as a lower direction.

Each of the attachment portions 2 includes an electrode 21, an insulator 22, a connection portion 23, a seal portion 24, and the like. On the other hand, the main body 3 includes a housing 31, a calculation unit 32, and the like. Two connection portions 33 protruding from a lower surface of the housing 31 are fixed to the main body 3. The connection portion 33 (first connection portion) on the main body 3 side and the connection portion 23 (second connection portion) on the attachment portion 2 side constitute a snap connection portion 10 attachable to and detachable from each other. The connection portion 33 has a male terminal 331, and the connection portion 23 has a female terminal 231. As the male terminal 331 having a rod shape is fitted into the female terminal 231, the male terminal 331 and the female terminal 231 are electrically connected in an easily detachable state.

As described above, the biological signal transmission device 1 according to the present embodiment includes two of the electrodes 21 and two of the snap connection portions 10. One of the electrodes 21 is electrically connected to each of the snap connection portions 10. Each of two of the insulators 22 is individually fixed to each of the connection portions 23 located on the electrode 21 side of each of the snap connection portions 10.

### 2-1. Configuration of attachment portion

The electrode 21 is, for example, a sheet-like member having adhesiveness, and is attached to a surface S of a living body with an easily peelable adhesive force. The electrode 21 is formed of, for example, gel-like conductive resin. In this example, the electrode 21 is formed in a circular shape, but is not limited to a circular shape, and may have another shape such as a rectangular shape. Further, the electrode 21 can be formed of, for example, silver paste, but is not limited to silver paste, and can be formed of various other materials having conductivity.

The insulator 22 is, for example, a sheet-like member having insulating property and waterproof property. In this example, the insulator 22 is formed in a circular shape having a larger diameter than the electrode 21. However, the insulator 22 is not limited to a circular shape, and may have another shape such as a rectangular shape. A recessed portion 221 is formed on a lower surface of the insulator 22, and the electrode 21 is fixed in a state of being accommodated in the recessed portion 221. A lower surface of the electrode 21 accommodated in the recessed portion 221 is flush with the lower surface of the insulator 22 or protrudes from the lower surface of the insulator 22. The insulator 22 can be formed of, for example, insulating resin such as urethane resin or silicone resin, but is not limited to these, and can be formed of various other materials having insulating property and waterproof property.

The connection portion 23 is electrically connected to the electrode 21. The connection portion 23 has a female terminal 231 having an annular shape formed of, for example, conductive metal. A lower surface of the female terminal 231 abuts on an upper surface of the electrode 21. On an upper surface of the female terminal 231, a recessed portion 232 for removably receiving the male terminal 331 of the connection portion 33 on the main body 3 side is formed. The upper surface of the female terminal 231 constitutes a receiving surface 230. In the recessed portion 232 of the female terminal 231, the male terminal 331 is received from the tip, and in this state, the connection portion 23 is electrically connected to the connection portion 33.

The seal portion 24 is a tubular member that covers an outer periphery of the female terminal 231. The seal portion 24 has flexibility and waterproof property. Alower end surface of the seal portion 24 is fixed to an upper surface of the insulator 22. A protruding amount of the seal portion 24 with respect to the upper surface of the insulator 22 is larger than a protruding amount of the female terminal 231 with respect to the upper surface of the insulator 22.

An opening 241 is formed on an upper end surface of the seal portion 24. The opening 241 has, for example, a circular shape, and has an inner diameter smaller than an outer diameter of the female terminal 231 and larger than an inner diameter of the female terminal 231 (inner diameter of the recessed portion 232). By the above, the recessed portion 232 of the female terminal 231 is exposed to the outside through the opening 241.

The seal portion 24 may have any configuration as long as the female terminal 231 is accommodated in the inside and the recessed portion 232 of the female terminal 231 can be exposed to the outside. Therefore, the seal portion 24 is not limited to a cylindrical shape, and may have another optional shape. The seal portion 24 can be formed of, for example, insulating resin such as urethane resin or silicone resin, but is not limited to these, and can be formed of various other materials having insulating property and waterproof property.

### 2-2. Configuration of main body

The housing 31 of the main body 3 is a hard hollow member in which various components such as an electronic component are accommodated. The housing 31 can be formed of, for example, insulating resin such as polycarbonate resin or ABS resin, but is not limited to insulating resin, and can be formed of various other materials having insulating property and waterproof property.

In the present embodiment, the housing 31 is composed of a member having a substantially rectangular shape in plan view. On a lower surface of the housing 31, two of the male terminals 331 are arranged side by side along a longitudinal direction of the housing 31, and each of the male terminals 331 protrudes in a lower direction. A distance between two of the male terminals 331 substantially coincides with a distance between the recessed portions 232 of two of the female terminals 231. Therefore, by bringing the main body 3 closer to two of the attachment portions 2, each of the male terminals 331 can be inserted into the recessed portion 232 of a corresponding one of the female terminals 231 at a time, and electrical connection by two of the snap connection portions 10 can be simultaneously performed.

The calculation unit 32 includes a processor such as a central processing unit (CPU), for example. The calculation unit 32 is accommodated in the housing 31 and is electrically connected to each of the male terminals 331 via wiring (not illustrated). The calculation unit 32 processes a signal input from each of the electrodes 21 via the snap connection portion 10. Processing of a signal by the calculation unit 32 includes various types of arithmetic processing for obtaining an electrocardiogram, an electromyogram, an electroencephalogram, or the like on the basis of the signal.

### 2-3. Specific configuration of snap connection portion

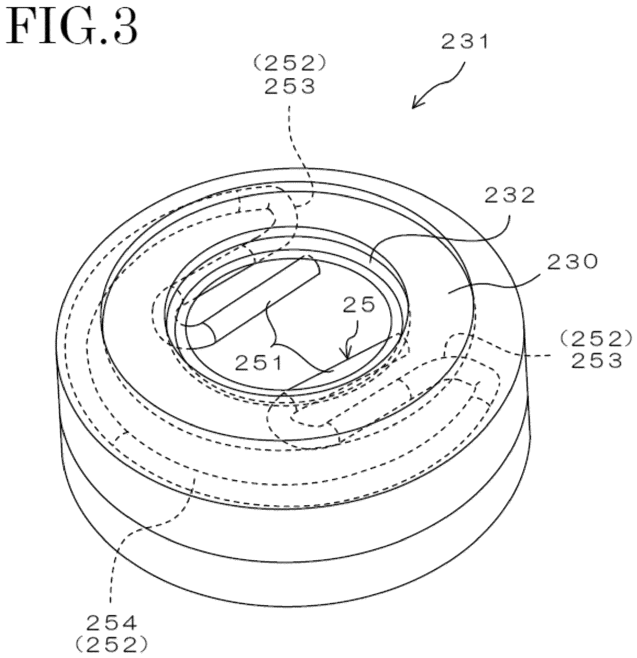

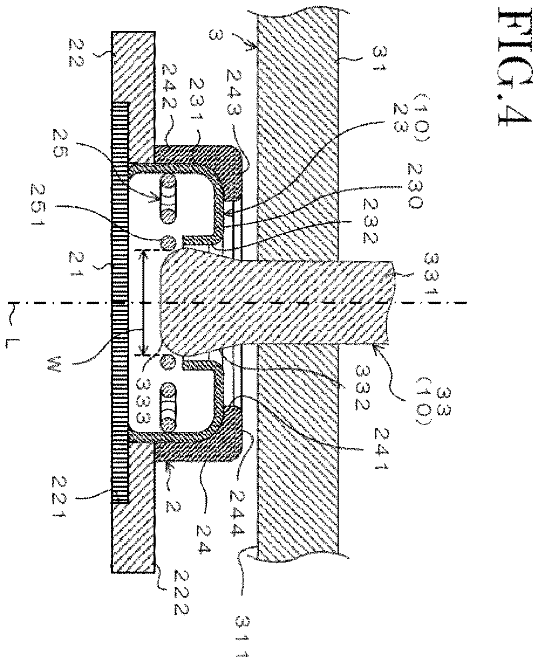

FIG. 3 is a perspective view of the female terminal 231. FIG. 4 is a cross-sectional view around the snap connection portion 10. Note that FIG. 4 illustrates a state in the middle of insertion of the male terminal 331 into the recessed portion 232 of the female terminal 231.

The female terminal 231 is a hollow member made from metal and has conductivity. The female terminal 231 protrudes in an upper direction from an upper surface of the insulator 22. That is, the upper surface of the insulator 22 constitutes a first fixing surface 222 having insulating property fixed to the electrode 21 side (female terminal 231 side) of the snap connection portion 10. The insulator 22 constitutes a first insulator having the first fixing surface 222.

Inside the female terminal 231, a biasing member 25 for making electrical connection with the male terminal 331 is provided. The biasing member 25 is, for example, a spring-like member formed by bending or curving one metal wire, and has conductivity. Specifically, the biasing member 25 includes a pair of contact portions 251 extending substantially parallel to each other at a constant interval, and a coupling portion 252 coupling a pair of the contact portions 251.

The coupling portion 252 includes a pair of spring portions 253 extending in an S shape from one end of each of the contact portions 251, and a fixing portion 254 that fixes the spring portion 253 to the female terminal 231. The biasing member 25 can change a distance between a pair of the contact portions 251 by elastic deformation in a pair of the spring portions 253. The fixing portion 254 is formed in an arc shape having a curvature corresponding to an inner peripheral surface of the female terminal 231. The fixing portion 254 is fixed in a state of abutting on the inner peripheral surface of the female terminal 231, so that the female terminal 231 and the biasing member 25 are electrically connected.

An interval W between a pair of the contact portions 251 is smaller than an inner diameter of the recessed portion 232. In a state where the biasing member 25 is fixed in the female terminal 231, a pair of the contact portions 251 are located in a lower direction of the recessed portion 232 and is exposed to the inner side than the recessed portion 232 (on a central axis L side of the female terminal 231) in plan view By the above, when a tip portion of the male terminal 331 is inserted into the recessed portion 232 from an upper direction as illustrated in FIG. 4, the tip portion of the male terminal 331 comes into contact with a pair of the contact portions 251.

The male terminal 331 is a metal member extending in a vertical direction and has conductivity. The male terminal 331 protrudes in a lower direction from a lower surface of the housing 31 of the main body 3. That is, the lower surface of the housing 31 of the main body 3 constitutes a second fixing surface 311 having insulating property fixed to the side (the male terminal 331 side) opposite to the electrode 21 side of the snap connection portion 10. The housing 31 constitutes a second insulator having the second fixing surface 311.

A tapered surface 332 having a truncated cone shape formed to expand in a lower direction is formed at a lower end portion of the male terminal 331. The interval W between a pair of the contact portions 251 is smaller than an outer diameter of a lower end of the tapered surface 332 and larger than an outer diameter of an upper end of the tapered surface 332. That is, the interval W between a pair of the contact portions 251 coincides with an outer diameter in an intermediate portion of the tapered surface 332.

The lower end of the tapered surface 332 is continuous with a lower end surface of the male terminal 331 via a smooth curved surface 333. The interval W between a pair of the contact portions 251 is equal to an outer diameter of an intermediate portion of the curved surface 333. Therefore, when a tip portion of the male terminal 331 is inserted into the recessed portion 232 from an upper direction as illustrated in FIG. 4, first, a pair of the contact portions 251 come into contact with the curved surface 333. From this state, when the male terminal 331 is further pushed into the recessed portion 232, the biasing member 25 is elastically deformed, and a pair of the contact portions 251 are separated by the curved surface 333.

Then, when the male terminal 331 is further pushed into the recessed portion 232, a pair of the contact portions 251 climb over the curved surface 333 and come into contact with the tapered surface 332. Since the tapered surface 332 is tapered in an upper direction, an interval between a pair of the contact portions 251 becomes closer to the initial interval W while the contact portions 251 slide on the tapered surface 332. In this way, the male terminal 331 and the female terminal 231 are fitted at the position where a pair of the contact portions 251 are in contact with an intermediate portion of the tapered surface 332, and snap connection is achieved.

However, the biasing member 25 is not limited to the above-described shape, and may have any configuration as long as the biasing member 25 applies a biasing force to the tapered surface 332 of the male terminal 331 received in the recessed portion 232.

### 2-4. Specific configuration of seal portion

As illustrated in FIG. 4, the seal portion 24 includes a peripheral wall 242 having a tubular shape and an extension portion 243 having an annular shape extending from an upper end surface of the peripheral wall 242 toward the central axis L side. The peripheral wall 242 and the extension portion 243 are integrally formed, and an inner surface shape of them substantially matches an outer surface shape of the female terminal 231. The seal portion 24 watertightly covers an outer periphery of the female terminal 231 in a state where an inner surface of the seal portion 24 is in close contact with a part of an upper surface and an outer peripheral surface of the female terminal 231. The seal portion 24 may be bonded to, or cover without being bonded to the female terminal 231.

The opening 241 described above is formed at a central portion of the extension portion 243. The extension portion 243 extends on the receiving surface 230 of the female terminal 231 and annularly surrounds a periphery of the recessed portion 232. An upper surface of the extension portion 243 constitutes a contact surface 244 formed of a flat surface. The second fixing surface 311 facing the extension portion 243 in an upper direction is also formed of a flat surface, and the contact surface 244 of the seal portion 24 can be brought into close contact with the second fixing surface 311.

### 3. Aspect of snap connection

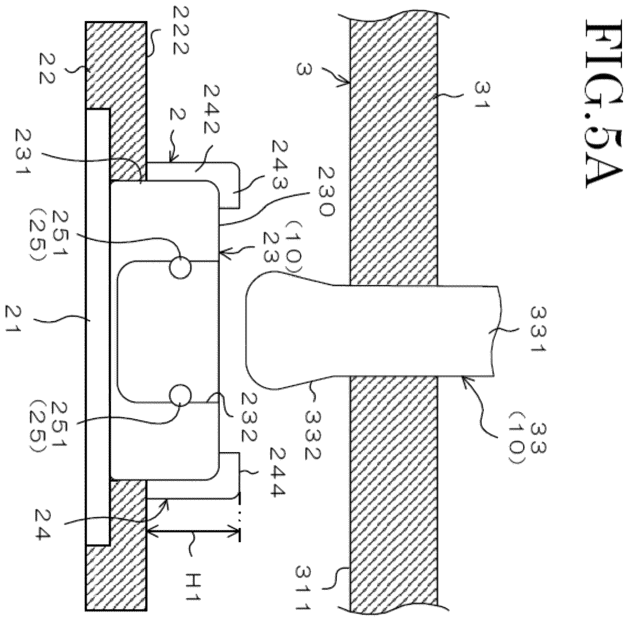

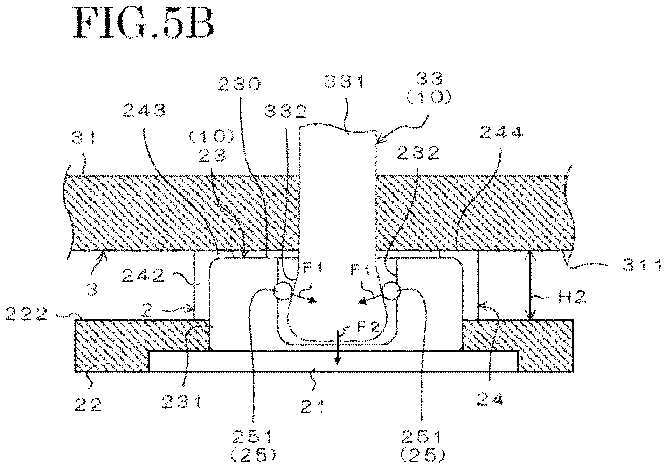

FIGS. 5A and 5B are schematic cross-sectional views for explaining an aspect of snap connection by the snap connection portion 10. FIG. 5Aillustrates a state before two of the connection portions 23 and 33 are electrically connected. FIG. 5B illustrates a state in which two of connection portions 23 and 33 are electrically connected.

When snap connection is performed by the snap connection portion 10, first, as illustrated in FIG. 5A, two of the connection portions 23 and 33 are in a state of facing each other. In this state, the seal portion 24 is not in contact with the second fixing surface 311 of the main body 3, and a height H1 of the seal portion 24 is a height in a normal state in which the seal portion 24 is not elastically deformed.

When two of the connection portions 23 and 33 are brought close to each other from the state of FIG. 5A, a tip portion of the male terminal 331 is received in the recessed portion 232 of the female terminal 231 and comes into contact with a pair of the contact portions 251 of the biasing member 25. After the above, when the male terminal 331 is further pushed into the recessed portion 232, a pair of the contact portions 251 are in a state of being in contact with the tapered surface 332 as illustrated in FIG. 5B, and two of the connection portions 23 and 33 are snap-connected. At this time, the contact surface 244 of the seal portion 24 is in close contact with the second fixing surface 311 of the main body 3, and the seal portion 24 is in a state of being arranged between the first fixing surface 222 and the second fixing surface 311.

In the state of FIG. 5B, a biasing force F1 acts on the tapered surface 332 from a pair of the contact portions 251. Since a contact surface of the male terminal 331 with respect to a pair of the contact portions 251 is the tapered surface 332, a force F2 for drawing into the recessed portion 232 acts on the male terminal 331 by the biasing force F1 from a pair of the contact portions 251. The force F2 acts in a direction in which the male terminal 331 and the female terminal 231 are moved closer to each other.

A height H2 of the second fixing surface 311 with respect to the first fixing surface 222 in the state of FIG. 5B is lower than the height H1 of the seal portion 24 in the state of FIG. 5A. Therefore, in a state where the male terminal 331 is received in the female terminal 231 as illustrated in FIG. 5B, the seal portion 24 is compressed by being sandwiched between the first fixing surface 222 and the second fixing surface 311. At an upper end portion of the seal portion 24, the extension portion 243 is compressed by being sandwiched between the receiving surface 230 and the second fixing surface 311.

At this time, a periphery of the male terminal 331 and the female terminal 231 becomes in a state of being sealed by the seal portion 24, and waterproof property of a contact portion between the male terminal 331 and the female terminal 231 is secured. That is, even in a case where liquid exists between the first fixing surface 222 and the second fixing surface 311, it is possible to prevent the liquid from entering a contact portion between the male terminal 331 and the female terminal 231.

When two of the connection portions 23 and 33 are moved away from the state of FIG. 5B, a tip portion of the male terminal 331 is removed from the inside of the recessed portion 232 of the female terminal 231 against the biasing force F1 of the biasing member 25, and the state of FIG. 5A is obtained again. In this state, the second fixing surface 311 of the main body 3 is in a non-contact state with the contact surface 244 of the seal portion 24, so that the sealed state by the seal portion 24 is released.

### 4. Variation of insulator

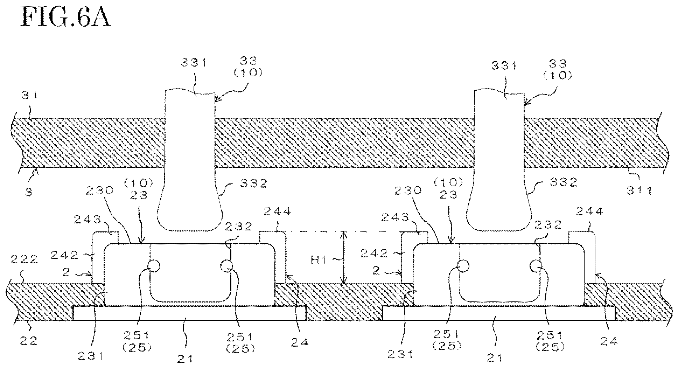

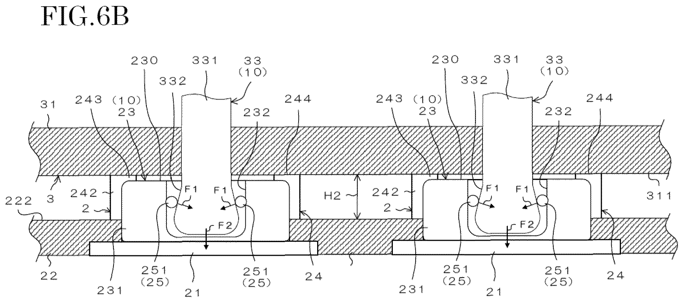

FIGS. 6A and 6B are schematic cross-sectional views for explaining a variation of the insulator 22. FIG. 6Aillustrates a state before two of the connection portions 23 and 33 are electrically connected. FIG. 6B illustrates a state in which two of the connection portions 23 and 33 are electrically connected. Note that, in FIGS. 6A and 6B, the same configurations as those in the above embodiment are denoted by the same reference numerals in the diagram, and detailed description of the configurations will be omitted.

In this variation, each of the connection portions 23 positioned on the electrode 21 side of each of the snap connection portions 10 is not individually fixed to two of the insulators 22, but is fixed to one of the insulator 22. An upper surface of the insulator 22 constitutes the first fixing surface 222, and the connection portions 23 are fixed to the common first fixing surface 222. Further, the connection portion 33 of each of the snap connection portions 10 is fixed to the common second fixing surface 311 of the main body 3.

An outer periphery of the female terminal 231 of each of the connection portions 33 is covered with an individual one of the seal portion 24. The shape of each of the seal portions 24 is the same. Therefore, in a normal state where the seal portion 24 is not elastically deformed as illustrated in FIG. 6A, the heights H1 of the seal portions 24 coincide with each other. When the first fixing surface 222 and the second fixing surface 311 are brought close to each other from the state of FIG. 6A and each of the male terminals 331 is received in the recessed portion 232 of a corresponding one of the female terminals 231 as illustrated in FIG. 6B, the biasing force F1 acts on each of the male terminals 331 from the biasing member 25 provided in each of the female terminals 231.

The height H2 of the second fixing surface 311 with respect to the first fixing surface 222 in the state of FIG. 6B is lower than the height H1 of the seal portions 24 in the state of FIG. 6A. Therefore, in a state where a corresponding one of the male terminals 331 is received in each of the female terminals 231 as illustrated in FIG. 6B, each of the seal portions 24 is compressed by being sandwiched between the first fixing surface 222 and the second fixing surface 311. At an upper end portion of each of the seal portions 24, the extension portion 243 is compressed by being sandwiched between the receiving surface 230 and the second fixing surface 311.

At this time, a periphery of each of the male terminals 331 and each of the female terminals 231 becomes in a state of being sealed by the seal portion 24, and waterproof property of a contact portion between each of the male terminals 331 and each of the female terminals 231 is secured. That is, even in a case where liquid exists between the first fixing surface 222 and the second fixing surface 311, it is possible to prevent the liquid from entering a contact portion between the male terminal 331 and the female terminal 231.

However, the seal portion 24 is not limited to the configuration in which two of the seal portions 24 are provided in a manner corresponding to the female terminals 231, and may have a configuration in which an outer periphery of the female terminals 231 is covered with the common seal portion 24. That is, two of the seal portions 24 in FIGS. 6A and 6B may be configured to be connected to each other.

### 5. Variation of seal portion

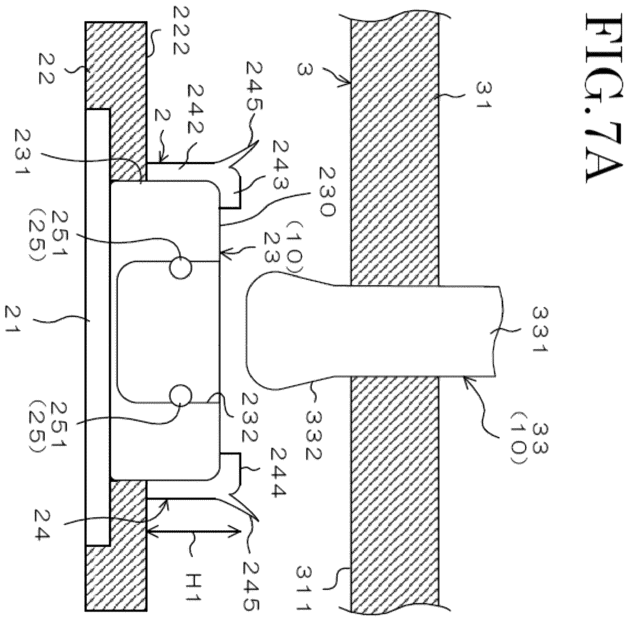

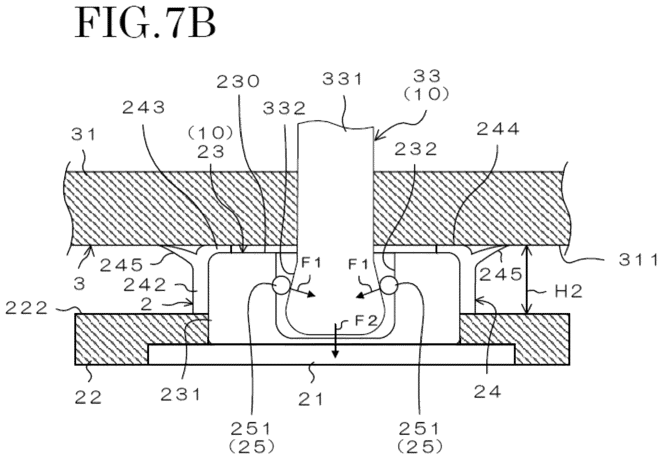

FIGS. 7A and 7B are schematic cross-sectional views for explaining a first variation of the seal portion 24. FIG. 7A illustrates a state before two of the connection portions 23 and 33 are electrically connected. FIG. 7B illustrates a state in which two of the connection portions 23 and 33 are electrically connected. Note that, in FIGS. 7A and 7B, the same configurations as those in the above embodiment are denoted by the same reference numerals in the diagram, and detailed description of the configurations will be omitted.

In this variation, a sucker portion 245 is formed so as to protrude from a part of each of the seal portions 24. The sucker portion 245 may protrude from the peripheral wall 242 or may protrude from the extension portion 243. The sucker portion 245 is an annular member formed integrally with the seal portion 24, and has flexibility and waterproof property. An inner diameter of the sucker portion 245 is larger than the opening 241 formed on an upper end surface of the seal portion 24.

The sucker portion 245 extends in a direction intersecting a direction in which the extension portion 243 extends. Specifically, the sucker portion 245 is formed in a truncated cone shape so as to expand with increasing distance from the first fixing surface 222. An angle of a direction in which the sucker portion 245 extends with respect to the direction in which extension portion 243 extends is larger than 90° and smaller than 180°, for example. The angle may be preferably 120° to 150°, and more preferably 130° to 140°.

The sucker portion 245 has a cross-sectional shape tapered toward a tip portion. The tip portion of the sucker portion 245 extends to a position farther from the first fixing surface 222 than the contact surface 244 of the seal portion 24. Therefore, in a case where two of the connection portions 23 and 33 are brought close to each other from the state where two of the connection portions 23 and 33 face each other as illustrated in FIG. 7A, the tip portion of the sucker portion 245 comes into contact with the second fixing surface 311 of the main body 3 before the contact surface 244.

When two of the connection portions 23 and 33 are further brought close to each other, the tip portion of the sucker portion 245 is expanded by the second fixing surface 311. Then, in a state where a tip portion of the male terminal 331 is received in the recessed portion 232 of the female terminal 231 as illustrated in FIG. 7B, air inside the sucker portion 245 is pushed out, so that the sucker portion 245 is adhered to the second fixing surface 311. In the state of FIG. 7B, at an upper end portion of the seal portion 24, the extension portion 243 is compressed by being sandwiched between the receiving surface 230 and the second fixing surface 311. Therefore, a periphery of the male terminal 331 and the female terminal 231 is sealed at two positions of the extension portion 243 and the sucker portion 245.

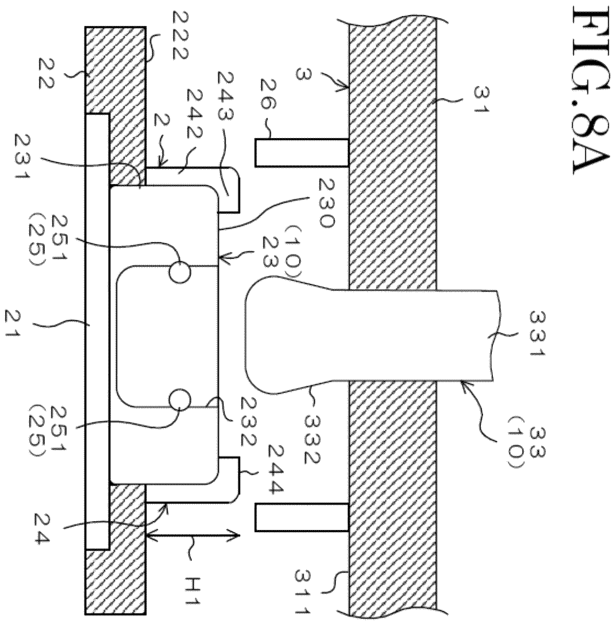

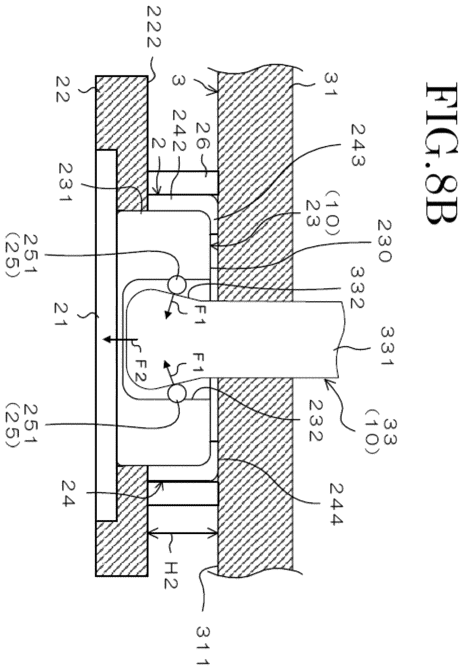

FIGS. 8A and 8B are schematic cross-sectional views for explaining a second variation of the seal portion 24. FIG. 8A illustrates a state before two of the connection portions 23 and 33 are electrically connected. FIG. 8B illustrates a state in which two of the connection portions 23 and 33 are electrically connected. Note that, in FIGS. 8A and 8B, the same configurations as those in the above embodiment are denoted by the same reference numerals in the diagram, and detailed description of the configurations will be omitted.

In this variation, a seal portion 26 fixed to a lower surface of the housing 31 is provided separately from the seal portion 24 fixed to an upper surface of the insulator 22. The seal portion 26 is a tubular member, and is arranged at an interval around the male terminal 331 on the same axis. An inner diameter of the seal portion 26 is equal to or more than an outer diameter of the seal portion 24, and is equal to the outer diameter of the seal portion 24 in this example. A height of the seal portion 26 may be the same as the height H1 of the seal portion 24. The seal portion 26 can be formed of, for example, insulating resin such as urethane resin or silicone resin, but is not limited to these, and can be formed of various other materials having insulating property and waterproof property. A material of the seal portion 26 may be the same as or different from a material of the seal portion 24. Further, the seal portion 26 is not limited to a cylindrical shape, and may have another optional shape.

When two of the connection portions 23 and 33 are brought close to each other from the state of FIG. 8A, a tip portion of the male terminal 331 is received in the recessed portion 232 of the female terminal 231 as in FIG. 8B. At this time, the seal portion 24 enters the inside of the seal portion 26, and each of the seal portion 24 and the seal portion 26 is compressed by being sandwiched between the first fixing surface 222 and the second fixing surface 311. By the above, a periphery of the male terminal 331 and the female terminal 231 becomes in a state of being sealed by the seal portion 24 and the seal portion 26, and waterproof property of a contact portion between the male terminal 331 and the female terminal 231 is secured.

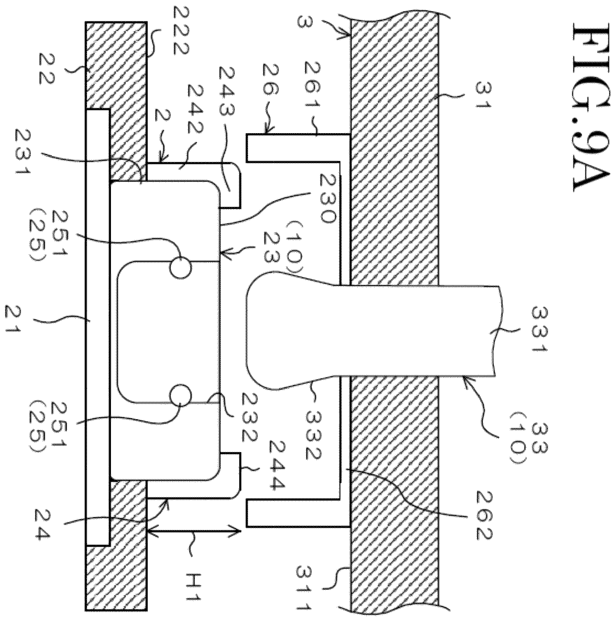

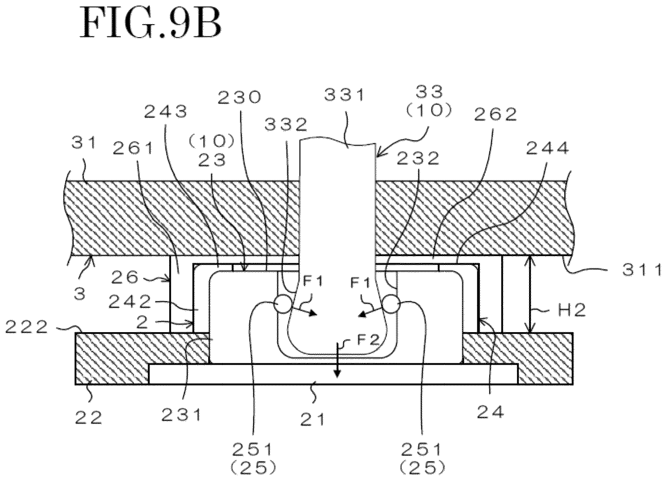

FIGS. 9A and 9B are schematic cross-sectional views for explaining a third variation of the seal portion 24. FIG. 9A illustrates a state before two of the connection portions 23 and 33 are electrically connected. FIG. 9B illustrates a state in which two of the connection portions 23 and 33 are electrically connected. Note that, in FIGS. 9A and 9B, the same configurations as those in the above embodiment are denoted by the same reference numerals in the diagram, and detailed description of the configurations will be omitted.

In this variation, the seal portion 26 has a configuration different from that of the second variation. Specifically, the seal portion 26 includes a tubular portion 261 and an end surface portion 262. The tubular portion 261 is a tubular member, and is arranged at an interval around the male terminal 331 on the same axis. An inner diameter of the tubular portion 261 is equal to or more than an outer diameter of the seal portion 24, and is equal to the outer diameter of the seal portion 24 in this example. A height of the tubular portion 261 may be the same as the height H1 of the seal portion 24.

The end surface portion 262 is fixed in a manner abutting on a lower surface of the housing 31 and closes an upper end surface of the tubular portion 261. The seal portion 26 is positioned with respect to the male terminal 331 as the male terminal 331 penetrates a central portion of the end surface portion 262. By the above, it is possible to prevent the seal portion 26 from being displaced with respect to the male terminal 331 and to improve relative positional accuracy between the seal portion 24 and the seal portion 26 when a tip portion of the male terminal 331 is received in the recessed portion 232 of the female terminal 231. When the seal portion 24 enters the inside of the seal portion 26 as illustrated in FIG. 9B from the state of FIG. 9A, the end surface portion 262 of the seal portion 26 abuts on and is in close contact with an upper end surface of the seal portion 24, so that waterproofness of a contact portion between the male terminal 331 and the female terminal 231 is further improved.

### 6. Another variation

The seal portion 24 may be fixed to the second fixing surface 311 instead of the first fixing surface 222. Alternatively, the seal portion 24 may have a configuration in which a first seal portion protruding in an upper direction from the first fixing surface 222 and a second seal portion protruding in a lower direction from the second fixing surface 311 are included, and tip portions of the first seal portion and the second seal portion abut on each other and are compressed by each other. In this case, the configuration may be such that the first seal portion is integrally formed with the first fixing surface 222, and the second seal portion is integrally formed with the second fixing surface 311.

The configuration of the seal portion 24 is not limited to the configuration of being fixed to at least one of the first fixing surface 222 and the second fixing surface 311. That is, the seal portion 24 may be separated from the first fixing surface 222 and the second fixing surface 311. In this case, the seal portion 24 may be configured to be compressed as the male terminal 331 and the female terminal 231 are fitted to each other after the seal portion 24 is arranged between the first fixing surface 222 and the second fixing surface 311.

The seal portion 24 does not need to be in contact with the snap connection portion 10. That is, the seal portion 24 may be configured to be arranged with respect to the snap connection portion 10 with a space between them. In this case, the seal portion 24 may be formed of a material having no insulating property.

Further, the seal portion 24 may be formed, for example, by being applied to at least one of the first fixing surface 222 and the second fixing surface 311 instead of being formed in advance as a tubular member.

The electrode 21 and the female terminal 231 may be electrically connected by wire or wirelessly, for example, without limitation to the configuration of being electrically connected by direct contact. Further, the male terminal 331 may be electrically connected to the main body 3 by wire or wirelessly, for example, without limitation to the configuration of being directly attached to the main body 3.

The snap connection portion 10 is not limited to the configuration in which the connection portion 23 on the electrode 21 side has the female terminal 231 and the connection portion 33 on the side opposite to the electrode 21 side has the male terminal 331. That is, in the snap connection portion 10, the connection portion 23 on the electrode 21 side may have the male terminal 331, and the connection portion 33 on the opposite side to the electrode 21 side may have the female terminal 231.

Note that the number of the attachment portions 2 to be attached to and detached from a living body is not limited to two, and only one of the attachment portion 2 may be provided, or three or more of the attachment portions 2 may be provided.

### 7. Second embodiment

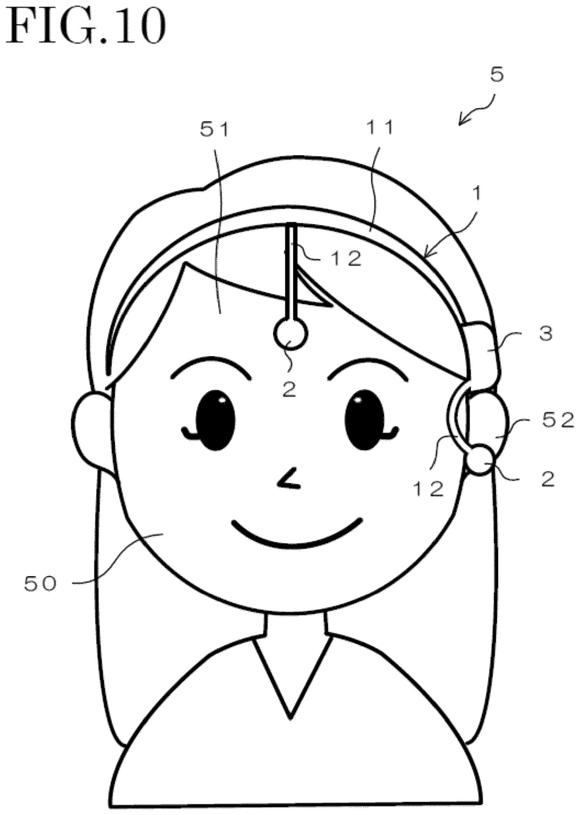

FIG. 10 is a schematic diagram illustrating a configuration example of the biological signal transmission device 1 according to a second embodiment. In the above embodiment, the description has been made on the configuration in which the main body 3 is attached to the attachment portion 2 so that the male terminal 331 and the female terminal 231 are electrically connected. On the other hand, in the present embodiment, the attachment portion 2 and the main body 3 are electrically connected via a wiring 12. In this case, the attachment portion 2 is provided with the male terminal 331 and the female terminal 231, and similar snap connection to that in the above embodiment is performed in the attachment portion 2.

In the present embodiment, since the attachment portion 2 and the main body 3 are electrically connected via the wiring 12, the degree of freedom in an attachment position of the attachment portion 2 with respect to a living body 5 is high. The attachment portion 2 can be attached to an optional position of a skin 50 of the living body 5. For example, in a case of measuring a brain wave, as illustrated in FIG. 10, two of the attachment portions 2 are provided and attached to a forehead 51 and an ear 52.

In this example, a fixture 11 for fixing the main body 3 to the living body 5 is provided. The main body 3 is attached to the fixture 11. Apart of the wiring 12 may be integrally held by the fixture 11.

### 8. Aspect

It is understood by those skilled in the art that a plurality of the exemplary embodiments described above are specific examples of an aspect below.

(Clause 1) A biological signal transmission device according to an aspect includes:
an electrode attached to a living body;
a snap connection portion electrically connected to the electrode;
a first insulator having a first fixing surface fixed to the electrode side of the snap connection portion;
a second insulator having a second fixing surface fixed to a side opposite to the electrode side of the snap connection portion; and
a seal portion arranged between the first fixing surface and the second fixing surface.

The snap connection portion may include:
a first connection portion having a male or protruding terminal on which a tapered surface is formed;
a second connection portion having a female or recessed terminal for removably receiving the male terminal, the second connection portion being electrically connected to the first connection portion in a state where the male terminal is received in the female terminal; and
a biasing member that comes into contact with the tapered surface of the male terminal received in the female terminal and is arranged to apply a force in a direction in which the male terminal and the female terminal are moved closer to each other, and
the seal portion may be configured to:
   seal a periphery of the male terminal and the female terminal with being sandwiched and compressed between the first fixing surface and the second fixing surface in response to the reception of the male terminal into the female terminal; and
   release the sealing state in response to the removal of the male terminal out of the female terminal.

According to the biological signal transmission device described in Clause 1, in a state where the snap connection portion has the male terminal and the female terminal, and the male terminal is received in the female terminal, the seal portion is compressed by being sandwiched between the first fixing surface and the second fixing surface, and a periphery of the male terminal and the female terminal is in a state of being sealed by the seal portion. This makes it possible to prevent water from entering a connection portion between the male terminal and the female terminal from the outside.

In particular, in a state where the male terminal is received in the female terminal, the biasing member comes into contact with the tapered surface of the male terminal and applies a force in a direction in which the male terminal and the female terminal are moved closer to each other. By the above, since the seal portion sandwiched between the first fixing surface and the second fixing surface can be further compressed, waterproof property of the connection portion between the male terminal and the female terminal can be effectively improved.

(Clause 2) In the biological signal transmission device described in Clause 1,
the female terminal may have a recessed portion that receives the male terminal, and a receiving surface on which the recessed portion is formed, and
the seal portion may have an extension portion extending on the receiving surface of the female terminal, and be configured such that the extension portion is compressed in a state where the male terminal is received in the female terminal.

According to the biological signal transmission device described in Clause 2, in a state where the male terminal is received in the female terminal, the extension portion of the female terminal extending on the receiving surface is in a compressed state, so that waterproof property of the connection portion between the male terminal and the female terminal can be further effectively improved.

(Clause 3) In the biological signal transmission device described in Clause 2,
the seal portion may have a sucker portion extending in a direction intersecting a direction in which the extension portion extends, and may be configured such that the sucker portion is adhered to the first fixing surface or the second fixing surface in a state where the male terminal is received in the female terminal.

According to the biological signal transmission device described in Clause 3, in a state where the male terminal is received in the female terminal, the sucker portion is in a state of being adhered to the first fixing surface or the second fixing surface, and thus waterproof property of the connection portion between the male terminal and the female terminal can be further effectively improved.

(Clause 4) In the biological signal transmission device described in any one of Clauses 1 to 3,
two of the electrodes and two of the snap connection portions may be included, and
the electrode may be electrically connected one by one to each of the snap connection portions.

According to the biological signal transmission device described in Clause 4, it is possible to prevent a short circuit from occurring between two of the electrodes and two of the snap connection portions.

(Clause 5) In the biological signal transmission device described in Clause 4,
two of the first insulators on each of which the first fixing surface is formed may be included, and
each of two of the first insulators may be individually fixed to the electrode side of each of the snap connection portions.

According to the biological signal transmission device described in Clause 5, the seal portion is sandwiched and compressed between the first fixing surface of two of the first insulators individually fixed to the electrode side of each of the snap connection portions and the second fixing surface, so that it is possible to prevent water from entering the connection portion between the male terminal and the female terminal of each of the snap connection portions from the outside.

(Clause 6) In the biological signal transmission device described in Clause 4,
one of the first insulator on which the first fixing surface is formed may be included, and
one of the first insulator may be fixed to the electrode side of each of the snap connection portions.

According to the biological signal transmission device described in Clause 6, the seal portion is sandwiched and compressed between the first fixing surface of the common first insulator fixed to the electrode side of each of the snap connection portions and the second fixing surface, so that it is possible to prevent water from entering the connection portion between the male terminal and the female terminal of each of the snap connection portions from the outside.

(Clause 7) In the biological signal transmission device described in any one of Clauses 1 to 6,
the biological signal transmission device further includes a main body including a calculation unit that processes a signal input from the electrode, and
the second insulator may be included in the main body.

According to the biological signal transmission device described in Clause 7, the seal portion is sandwiched and compressed between the first fixing surface and the second insulator included in the main body, so that it is possible to prevent water from entering the connection portion between the male terminal and the female terminal of the snap connection portion from the outside.

### DESCRIPTION OF REFERENCE SIGNS

- 1: biological signal transmission device
- 2: attachment portion
- 3: main body
- 4: holding member
- 5: living body
- 10: snap connection portion
- 21: electrode
- 22: insulator (first insulator)
- 23: connection portion
- 24: seal portion
- 25: biasing member
- 26: seal portion
- 31: housing (second insulator)
- 32: calculation unit
- 33: connection portion
- 221: recessed portion
- 222: first fixing surface
- 230: receiving surface
- 231: female terminal
- 232: recessed portion
- 241: opening
- 242: peripheral wall
- 243: extension portion
- 244: contact surface
- 311: second fixing surface
- 331: male terminal
- 332: tapered surface

## Claims

1. A biological signal transmission device comprising:
an electrode attached to a living body;
a snap connection portion electrically connected to the electrode;
a first insulator having a first fixing surface fixed to the electrode side of the snap connection portion;
a second insulator having a second fixing surface fixed to a side opposite to the electrode side of the snap connection portion; and
a seal portion arranged between the first fixing surface and the second fixing surface, wherein
the snap connection portion includes:
a first connection portion having a male terminal on which a tapered surface is formed;
a second connection portion having a female terminal for removably receiving the male terminal, the second connection portion being electrically connected to the first connection portion in a state where the male terminal is received in the female terminal; and
a biasing member that comes into contact with the tapered surface of the male terminal received in the female terminal and is arranged to apply a force in a direction in which the male terminal and the female terminal are moved closer to each other, and
the seal portion is configured to:
seal a periphery of the male terminal and the female terminal with being sandwiched and compressed between the first fixing surface and the second fixing surface in response to the reception of the male terminal into the female terminal; and
release the sealing state in response to the removal of the male terminal out of the female terminal.

2. The biological signal transmission device according to claim 1, wherein
the female terminal has a recessed portion that receives the male terminal, and a receiving surface on which the recessed portion is formed, and
the seal portion has an extension portion extending on the receiving surface of the female terminal, and is configured such that the extension portion is compressed in a state where the male terminal is received in the female terminal.

3. The biological signal transmission device according to claim 2, wherein
the seal portion has a sucker portion extending in a direction intersecting a direction in which the extension portion extends, and is configured such that the sucker portion is adhered to the first fixing surface or the second fixing surface in a state where the male terminal is received in the female terminal.

4. The biological signal transmission device according to claim 1, wherein
two of the electrodes and two of the snap connection portions are included, and
the electrode is electrically connected one by one to each of the snap connection portions.

5. The biological signal transmission device according to claim 4, wherein
two of the first insulators on each of which the first fixing surface is formed is included, and
each of two of the first insulators is individually fixed to the electrode side of each of the snap connection portions.

6. The biological signal transmission device according to claim 4, wherein
one of the first insulator on which the first fixing surface is formed is included, and
one of the first insulator is fixed to the electrode side of each of the snap connection portions.

7. The biological signal transmission device according to claim 1, further comprising:
a main body including a calculation unit that processes a signal input from the electrode, wherein
the second insulator is included in the main body.
